# EUROPEAN PATENT APPLICATION

(11) **EP 4 410 764 A1**
(43) Date of publication of application: **07.08.2024**
(21) Application number: 23155188.8
(22) Date of filing: 06.02.2023
(51) Int. Cl.: C07C 1/30, C07C 7/04, C07C 7/11, C07C 7/20, C07C 11/06, C07C 17/08, C07C 19/075

(54) **PROCESS FOR MITIGATING A REACTION OF HYDROGEN BROMIDE AND AN OLEFINICALLY UNSATURATED COMPOUND**

(71) Applicant: Sulzer Management AG, 8401 Winterthur (CH)
(72) Inventor: The designation of the inventor has not yet been filed
(74) Representative: Henkel & Partner mbB

(57) **Abstract**

The present invention relates to a process for mitigating a reaction of hydrogen bromide and optionally bromine with one or more olefinically unsaturated compounds comprising i) the step of adding hydrogen to a composition containing hydrogen bromide and olefinically unsaturated compound so as to obtain a composition containing hydrogen, hydrogen bromide and olefinically unsaturated compound and/or ii) the step of adding hydrogen to a precursor composition, from which a composition containing hydrogen, hydrogen bromide and olefinically unsaturated compound is formed.

## Description

The present invention relates to a process for mitigating a reaction of hydrogen bromide and olefinically unsaturated compound.

Olefins or alkenes, respectively, and particularly C₂₋₁₀-alkenes, such as ethylene and propylene, are valuable raw material for chemical syntheses and belong in fact to the most important starting materials in the petrochemical industry. For instance, ethylene is the starting material for producing polyethylene and propylene is the starting material for producing polypropylene, which are two of the most widely used polymers, since they are odorless, skin-friendly, physiologically harmless and have excellent mechanical properties. For example, polyethylene and polypropylene are used for packings, films, textiles, caps, medical products, mattress ticks and many other articles. Furthermore, ethylene and propylene are used for the production of other important chemicals, such as for producing ethylene glycol, propylene glycol, acrylonitrile, acrylic acid and propylene oxide.

C₂₋₁₀-alkenes are commonly produced by a direct catalytic dehydrogenation of the corresponding C₂₋₁₀-alkane, such as propylene by a direct catalytic dehydrogenation of propane. The catalytic dehydrogenation of propane to propylene is usually performed at a relatively high temperature of 550 to 680°C and at a comparable low-pressure, which is typically below atmospheric pressure. However, such processes based on the direct catalytic dehydrogenation of C₂₋₁₀-alkanes are - among others on account of the required high reaction temperature - energy intensive and ecologically harmful, if non-regenerable fuels, such as coal or the like, forming tremendous amounts of carbon dioxide during their combustion, are used to generate the required energy for maintaining the required high reaction temperature.

The predominantly used direct dehydrogenation processes utilize circulating catalysts or multiple reactors circulating in sequence production-purge-regeneration-purge. This requirement derives from the alkane-alkene equilibrium limitation, which is mitigated by the high temperature and/or low-pressure operation. All in all, these processes based on the direct catalytic dehydrogenation of a C₂₋₁₀-alkane have the drawback of high capital expenditures, of high operational costs and of producing high amounts of carbon dioxide.

In view of this, alternative processes for the production of olefinically unsaturated compounds, such as in particular C₂₋₁₀-alkenes, have been proposed. A promising alternative is a process comprising the step of brominating an alkane with bromine to a mixture comprising bromoalkane(s) and then dehydrobrominating the bromo-alkane(s) to a mixture of the corresponding alkene and hydrogen bromide, before the hydrogen bromide is separated from the alkene for instance by first distilling the mixture so as to separate heavies as bottom fraction therefrom and subjecting the overheads fraction containing hydrogen bromide and alkene to an absorption with water. In addition, the mixture comprising bromoalkene may also contain some unreacted bromine, which can be sent to the dehydrobromination step together with the bromoalkene(s). Moreover, in some cases, bromine may be formed also during regeneration of the dehydrobromination catalyst or different process upsets. The composition containing alkene and hydrogen bromide and optionally traces of bromine obtained during the dehydrobromination reaction is very sensitive to a rebromination reaction of the alkene with the hydrogen bromide or bromine. Such a rebromination is highly undesired because the formed (poly)alkylbromides need to be separated and recycled back to the dehydrobromination reaction or burnt in a thermal oxidizer to recover the bromine, which leads to a significant energy loss, to additional carbon dioxide emissions as well as to a reduction in the yield of the olefinically unsaturated compound. Therefore, a method for mitigating the rebromination of the reaction mixture obtained after the dehydrobromination step is very important for such a process.

Alkenes react very quickly in the cold with pure liquid bromine or with a solution of bromine in water or in an organic solvent. The double bond breaks and a bromine atom becomes attached to each carbon. The reaction is an example of electrophilic addition. The bromine is a very "polarizable" molecule and the approaching pi-bond in the ethene induces a dipole in the bromine molecule. However, the reaction in gas phase in case of a presence just a trace amount of bromine is not very fast and can be avoided. However, it is advantageous to neutralize the bromine before producing olefins.

The interaction of hydrogen bromide with olefinically unsaturated compounds is a free radical chain reaction. Typically, the presence of traces of oxygen or of a light dramatically increases the rate of this reaction. However, heavy compounds, such as aromatics, for instance benzene, toluene and xylols are mixtures thereof, may function as a radical trap so as to reduce the risk of a rebromination. However, the aromatic compound used as radical trap must be removed from the mixture, before the hydrogen bromide is separated from the olefinically unsaturated compound by water absorption, because otherwise the aromatic compound would contaminate the absorption medium so that the aromatic compound needs to be separated from the absorption medium involving high energy costs and capital expenditures, in order to allow to recycle the absorption medium. So, if the aromatic compound is removed from the mixture before the hydrogen bromide is separated from the olefinically unsaturated compound, the remaining mixture is again sensitive against rebromination of the olefinically unsaturated compound. Therefore, alternative mitigation measurements need to be found. It has been already proposed to reduce the temperature of the mixture containing hydrogen bromide and the olefinically unsaturated compound to less than 10°C down to even -80°C. However, this cooling consumes a lot of energy and creates significant technology issues for the water absorption step. Still alternatively, it was suggested to add sulfur-containing or oxygenated compounds as radical trap to the mixture containing hydrogen bromide and the olefinically unsaturated compound.

However, such a treatment leads to a pollution of the water used for the absorption step with water and thus requires further separation and purification steps, which increases the operational costs as well as the capital expenditures.

In view of this, the object underlying the present invention is to provide an efficient and economical process for mitigating a reaction of hydrogen bromide and optionally also of bromine with one or more olefinically unsaturated compounds in a composition containing these so as to protect the one or more olefinically unsaturated compounds from rebromination in particular in the subsequent separation step of separating the hydrogen bromide from the one or more olefinically unsaturated compounds.

In accordance with the present invention, this object is solved by providing a process for mitigating a reaction of hydrogen bromide and optionally bromine with one or more olefinically unsaturated compounds comprising i) the step of adding hydrogen to a composition containing hydrogen bromide and olefinically unsaturated compound so as to obtain a composition containing hydrogen, hydrogen bromide and olefinically unsaturated compound and/or ii) the step of adding hydrogen to a precursor composition, from which a composition containing hydrogen, hydrogen bromide and olefinically unsaturated compound is formed.

This solution bases on the finding that already the addition of small amounts of hydrogen to a composition containing hydrogen bromide and optionally also bromine with one or more olefinically unsaturated compounds reliably mitigates any rebromination of the olefinically unsaturated compound(s) by the hydrogen bromide and optionally bromine. Without being wished to be bound by any theory, it is considered that the effect of hydrogen is to neutralize any radicals in the composition and furthermore to reduce effects of oxide and salt effect of the walls of the device, through which the composition flows. In the case of bromine, the added hydrogen reacts very quickly with bromine in the gas phase resulting in hydrogen bromide formation, whereby all the traces of bromine are eliminated.

On account of the reliable mitigation of any rebromination of the olefinically unsaturated compound by the hydrogen bromide due to the addition of hydrogen, the resulting composition containing hydrogen, hydrogen bromide and olefinically unsaturated compound may be subjected to a separation step by absorption with an absorption medium, such as water or an aqueous solution, since the hydrogen may be easily separated from the olefinically unsaturated compound as well as from the absorbate. A further advantage is that the hydrogen efficiently reacts with traces of bromine being present in the absorption medium, which is recycled in the process. For instance, in the case that the absorption medium is recovered from the absorbate containing the absorption medium and hydrogen bromide by electrolysis, during which the hydrogen bromide is electrolyzed to hydrogen being obtained at one of the two electrodes and a mixture containing bromide, absorption medium and residues of hydrogen bromide (which was not converted during the electrolysis to hydrogen and bromide) being obtained at the other electrode, before the bromine is removed from the mixture of absorption medium for instance by distillation, residues of hydrogen bromide and traces of bromine obtained during the electrolysis remain in the absorption medium. Such remaining traces of bromine would, in the absence of hydrogen in the composition containing the hydrogen bromide and olefinically unsaturated compound, immediately lead to a rebromination of the olefinically unsaturated compound during the absorption step. All in all, the process in accordance with the present invention allows to efficiently and economically mitigate the reaction of hydrogen bromide and olefinically unsaturated compound in a composition containing both so as to protect the olefinically unsaturated compound from rebromination in particular in the subsequent separation step of separating the hydrogen bromide from the olefinically unsaturated compound.

Composition containing hydrogen, hydrogen bromide and olefinically unsaturated compound means in accordance with the present invention any composition containing hydrogen, hydrogen bromide and one or more olefinically unsaturated compounds. Likewise, composition containing hydrogen bromide and olefinically unsaturated compound means in accordance with the present invention any composition containing hydrogen bromide and one or more olefinically unsaturated compounds.

According to a preferred embodiment of the present invention, wherein the composition containing hydrogen, hydrogen bromide and olefinically unsaturated compound also comprises bromine and/or the precursor composition, from which a composition containing hydrogen, hydrogen bromide and olefinically unsaturated compound is formed, also comprises bromine.

In order to sufficiently mitigate a reaction of hydrogen bromide and olefinically unsaturated compound in the composition, it is suggested in a further development of the idea of the present invention to adjust the molar concentration of the hydrogen in the composition containing hydrogen, hydrogen bromide and olefinically unsaturated compound to at least 0.1% by mol. Preferably, the molar concentration of the hydrogen in the composition containing hydrogen, hydrogen bromide and olefinically unsaturated compound is adjusted to 0.2 to 90% by mol and more preferably to 0.5 to 60% by mol.

Good results are in particular obtained, when the molar ratio of hydrogen to olefinically unsaturated compound in the composition containing hydrogen, hydrogen bromide and olefinically unsaturated compound is adjusted to be 2:1 to 1:10.

Likewise, good results are in particular obtained, when the molar ratio of hydrogen to the hydrogen bromide in the composition containing hydrogen, hydrogen bromide and olefinically unsaturated compound is adjusted to be 2:1 to 1:5.

The present invention is not particularly limited concerning the kind of olefinically unsaturated compound. For instance, the olefinically unsaturated compound is an olefinically unsaturated hydrocarbon, more preferably a C₂₋₁₀-alkene, yet more preferably a C₂₋₅-monoalkene or a C₄₋₈-dialkene, still more preferably ethylene or propylene and most preferably propylene.

In accordance with the present invention, the hydrogen i) may be added to the already generated composition containing hydrogen bromide and olefinically unsaturated compound or ii) the hydrogen may be added before the composition containing hydrogen bromide and olefinically unsaturated compound is formed, namely to a precursor composition, from which the composition containing hydrogen, hydrogen bromide and olefinically unsaturated compound is formed, for instance by a dehydrobromination reaction of a bromoalkene.

In the first mentioned embodiment, in which the hydrogen is added to the already generated composition containing hydrogen bromide and olefinically unsaturated compound, it is preferred that the hydrogen is added to a composition containing 1 to 90% by weight and preferably 20 to 50% by weight of hydrogen bromide and 20 to 90% by weight and preferably 30 to 50% by weight of one or more olefinically unsaturated compounds. Such a composition may have been prepared, for example, by a dehydrobromination reaction as described in further detail below.

In the second mentioned embodiment, in which the hydrogen is added before the composition containing hydrogen bromide and olefinically unsaturated compound is formed, namely to a precursor composition, from which the composition containing hydrogen, hydrogen bromide and olefinically unsaturated compound is formed, for instance by a reaction, such as a dehydrobromination reaction, it is preferred that the hydrogen is added to a precursor composition, which comprises one or more brominated hydrocarbons, before the so obtained mixture is subjected to a dehydrobromination reaction so as to form the composition containing hydrogen, hydrogen bromide and one or more olefinically unsaturated compounds.

The aforementioned embodiment is not particularly limited concerning the kind of brominated hydrocarbon being contained in the precursor composition. Suitable examples for such brominated hydrocarbons are C₂₋₁₀-bromohydrocarbons, more preferably C₂₋₁₀-bromoalkanes, yet more preferably C₂₋₅-monobromoalkanes or C₄₋₈-dibromoalkanes, still more preferably bromoethane or bromopropane and most preferably bromopropane. Moreover, the brominated hydrocarbon may be a brominated alkene so as to dehydrobrominate it to a dialkane. Specific examples for suitable brominated hydrocarbons are monobromoethane, 1,2-dibromoethane, 1-bromo-propane, 2-bromopropane, 1,2-dipromopropane, 1,3-dibromopropane, 1-bromo-1-propene, 2-bromo-1-propene, 3-bromo-1-propene, monobromobutane, dibromobutane, monobromopentane, dibromopentane and arbitrary mixtures of two or more of the aforementioned compounds.

Good results are in particular obtained, when the precursor composition contains 10 to 99% by weight and preferably 20 to 75% by weight of one or more brominated hydrocarbons.

Preferably, the hydrogen is added to the precursor composition so that the molar ratio of the one or more brominated hydrocarbons and the hydrogen in the obtained mixture is 1:10 to 10:1 and preferably 1:5 to 5:1.

In a particular preferred embodiment of the present invention, the mixture, which is obtained by adding the hydrogen to the precursor composition, which comprises one or more brominated hydrocarbons, is subjected to a catalytic dehydrobromination reaction. Good results are in particular obtained, when the catalyst comprises a support material and thereon at least 200 ppm of at least one metal being selected from the group consisting of transition metals, noble metals and arbitrary combinations of two or more thereof. This solution bases on the finding that a catalyst comprising a support material and thereon at least 200 ppm of at least one metal being selected from the group consisting of transition metals, noble metals and arbitrary combinations of two or more thereof particularly efficiently catalyzes the dehydrobromination of bromohydrocarbons, such as of a C₂₋₁₀-bromoalkane, to the corresponding alkene, such as C₂₋₁₀-alkene. A further advantage of the aforementioned catalyst is that it is very stable.

Preferably the catalyst used for the catalytic dehydrobromination reaction comprises at least 75% by weight, more preferably least 75% by weight to 99.6% by weight, still more preferably least 85% by weight to 99.6% by weight and most preferably least 95% by weight to 99.6% by weight of support material.

In a further development of the idea of the present invention, it is suggested that the support material of the catalyst used for the catalytic dehydrobromination reaction has a specific surface area, measured by nitrogen adsorption, of at least 5 m²/g more, preferably of at least 20 m²/g and more preferably of at least 50 m²/g.

The present invention is not particularly limited concerning the chemical nature of the support material. Good results are, however, in particular obtained, when the support material is selected from the group consisting of aluminum oxides, silicon oxides, silica gels, silica-aluminas, zeolites, zirconias, titanias, niobias, silicon carbides, carbons, aluminophosphates molecular sieves, metalloaluminophosphate molecular sieves, amorphous aluminophosphates and arbitrary combinations of two or more thereof.

In accordance with a particularly preferred embodiment of the present invention, the support material of the catalyst is gamma-alumina oxide and/or boehmite.

Alumina of a high purity of 99.99% or even higher is preferably be synthesized by the alkoxide route. Alkoxide route is known production technology, which is based on hydrolysis of aluminum alkoxide. In this process, the alcohol(s) of the aluminum alkoxide is/are recycled back to the synthesis. The alumina is produced either as co-product with synthetic linear alcohols (Ziegler method) or directly from aluminum metal (on-purpose route). The Ziegler route for the synthesis of alumina involves the oligomerization of ethylene using triethylaluminium followed by oxidation. The triethylaluminium may be produced by reacting aluminium, ethylene and hydrogen gas. In the production process, two-thirds of the triethylaluminium produced is recycled back into the reactor and only one-third is used to produce the alcohol. The recycling step is used to produce triethylaluminium at a higher yield and in less time. Triethylaluminium reacts with ethylene to form higher molecular weight trialkylaluminium. The number of equivalents of ethylene n equals the total number of monomer units being grown on the initial ethylene chains, wherein n = x + y + z, wherein x, y, and z are the number of ethylene units per chain. Trialkylaluminium is oxidized with air to form aluminum alkoxides, and finally hydrolyzed to aluminum hydroxide and the desired alcohols.

1. AI+3 C₂H₄ +1.5 H₂→ Al(C₂H₅)₃

2. Al(C₂H₅)₃ + n ethylene -+ Al((CH₂CH₂)ₙCH₂CH₃)₃

3. Al((CH₂CH₂)ₙCH₂CH₃)₃+ O₂ → Al(O(CH₂CH₂)ₙCH₂CH₃)₃

4. Al(O(CH₂CH₂)ₙCH₂CH₃)₃ → Al(OH)₃ + CH₃CH₂(CH₂C₂)ₘOH

In accordance with another embodiment of the present invention, the alumina used as support material is selected from the group consisting of aluminum oxy-hydroxide, γ-alumina, boehmite, diaspore, transitional aluminas, ρ-alumina and arbitrary combinations of two or more thereof. Suitable examples for transitional aluminas are α-alumina, β-alumina, γ-alumina, δ-alumina, ε-alumina and κ-alumina, whereas suitable examples for ρ-alumina are aluminum trihydroxide, such as gibbsite, bayerite, nordstrandite and doyelite.

In accordance with an alternative embodiment of the present invention, the support material of the catalyst used for the catalytic dehydrobromination reaction is a silica. Suitable examples for sources of silica are silicates, precipitated silicas, for example, Zeosil range available from Rhodia, fumed silicas, for example, Aerosil-200 available from Degussa Inc., New York, N.Y., silicon compounds, such as tetraalkyl orthosilicates, for example, tetramethyl orthosilicate (TMOS) and tetraethylorthosilicate (TEOS), colloidal silicas or aqueous suspensions thereof, for example Ludox-HS-40 sol available from E.I. du Pont de Nemours, Wilmington, Del., silicic acid, alkali-metal silicate, or any combination thereof. Other suitable examples of amorphous silica include silica powders, such as Ultrasil VN3SP (commercially available from Degussa).

In accordance with a particular embodiment of the present invention, a silica sol or a combination of silica sol with precipitated or fumed silica is used as source of silicon for preparing the catalyst.

Also one or more zeolites may be used as support material of the catalyst for the catalytic dehydrobromination reaction. The zeolite may contain 0.01 to 10 wt% of phosphorus. Furthermore, the zeolite may be exchanged with Ca-, Mg-, La- and/or Ce-compounds or directly with the salts of the transition and/or noble metal(s) being provided on the support material of the catalyst to be used for the catalytic dehydrobromination reaction.

In accordance with an alternative embodiment of the present invention, a carbon is used as source of silicon for preparing the catalyst used for the catalytic dehydrobromination reaction. Preferably, the carbon is selected from graphite, carbon black, coke, petroleum coke and any arbitrary combination of two or more thereof. Particularly preferred is graphite.

In accordance with an alternative embodiment of the present invention, silicon carbide is used as source of silicon for preparing the catalyst used for the catalytic dehydrobromination reaction. Suitable examples therefore are sintered silicon carbide, nitride-bounded silicon carbide, recrystallised silicon carbide, reaction bonded silicon carbide and any arbitrary combination of two or more thereof.

In accordance with yet an alternative embodiment of the present invention, aluminophosphate (ALPO) and/or metalloaluminophosphate (MeAPO) is used as source of silicon for preparing the catalyst used for the catalytic dehydrobromination reaction.

As set out above, preferably the catalyst comprises a support material and thereon at least 200 ppm of at least one metal being selected from the group consisting of transition metals, noble metals and arbitrary combinations of two or more thereof. Preferably, the support material comprises therein 200 ppm to 20% by weight of one or more transition metals. The present invention is not particularly limited concerning the kind of transition metal. However, good results are in particular obtained, when the catalyst comprises as transition metal at least one compound being selected from the group consisting of iron, cobalt, nickel, molybdenum, manganese, lead, cupper, chromium, zinc, gallium and arbitrary combinations of two or more thereof.

Moreover, it is preferred that in the process a catalyst is used, which comprises a support material and thereon 200 ppm to 5% by weight of one or more noble metals. The one or more noble metals may be provided on the support material instead of one or more transition metals or in addition to one or more transition metals.

Also concerning the kind of noble metal, the present invention is not particularly limited. However, good results are in particular obtained, when the catalyst comprises as noble metal at least one compound being selected from the group consisting of silver, platinum, palladium, ruthenium, iridium and arbitrary combinations of two or more thereof.

The transition metal and/or noble metal may be introduced onto the support material in any known manner, such as by precipitation, by deposition, by impregnation, by ion exchange, by grafting, by anchoring, by chemical vapor deposition (CVD), by atomic layer epitaxy (ALE) or by encapsulation. Preferably, the transition metal and/or noble metal is introduced onto the support material by impregnation, because this technique allows to load a given porous support material with the transition metal and/or noble metal in a solid-state way (i.e. physical mixture of both component in solid state) or more preferably via wet impregnation (i.e. physical mixture of the support in solid state and the metal component dissolved in a liquid solution).

Before loading the transition metals and/or noble metal onto the support material, the support material is preferably shaped, which allows it to be processed to a catalyst having a desired granulometry and good crush strength. This is advantageous, because it allows to limit the pressure drop during the reaction and to prevent that the catalyst breaks down during the reaction into powder-like materials. For this purpose, the support material is preferably shaped, either alone or with addition of a binder. The binder may be selected to be resistant to the temperature and other conditions employed in the processes using the catalyst and the binder may be an inorganic material selected from the same chemical group as the material of the support material, namely silicas, metal silicates, zirconias, borates, aluminas, silica-aluminas, phosphates, for example amorphous aluminophosphates, calcium phosphates, clays, xonotlites, magnesias, metal oxides, such as zirconium dioxide, metals and gels including mixtures of silica and metal oxides. Clays are known to be essentially inert under a wide range of reaction conditions. Suitable clays include commercially available products such as kaolin, kaolinite, montmorillonite, attapulgite, saponite and bentonite. These clays may be used as mined in their natural state or they may also be employed in highly active forms, such as activated by an acid treatment procedure. Clays contribute to strength as a binder enhancing the attrition resistance properties of the catalyst particles, and clays in combination with other binders contributes to the hardness of the particles. Clays also start as small particles and have a higher density, such that when combined with the molecular sieve and binder provide for denser particles, imparting the desirable characteristic of higher density.

Once the support material is shaped, the transition metal and/or noble metal is loaded onto the support material and the catalyst is present in a substantially dry or dried state, preferably a heat treatment, for example calcination, is performed in order to harden and/to or activate the composition. Preferably, the heat treatment is carried out at a temperature of at least 400°C for a period of 1 to 48 hours. A calcination may be carried out, for example, in a rotary calciner, fluid bed calciner, or a batch oven.

In accordance with a further preferred embodiment of the present invention, the catalyst has a silicon dioxide content of less than 0.4% by weight, has a content of sulfur and sulfur containing compounds of less than 0.5% by weight and has a content of sodium and sodium containing compounds of less than 200 ppm.

Good results are in particular obtained, when the catalyst contains, in sum, alkali earth metals, rare earth metals, compounds containing at least one alkali earth metal and compounds containing at least rare earth metal in an amount of 0.1 % by weight to 5% by weight. Preferred alkali earth metals and rare earth metals are calcium, magnesium, yttrium and lanthanum.

Good results are in particular obtained, when the catalyst contains low amounts of or better even no alkali metals. In view of this, it is preferred that the catalyst contains, in sum, alkali metals and compounds containing at least one alkali metal in an amount of less than 1,000 ppm, preferably of less than 200 ppm, more preferably of less than 100 ppm.

In accordance with a further preferred embodiment of the present invention, the catalyst contains, in sum, chlorine in an amount of less than 1,000 ppm, preferably of less than 200 ppm, more preferably of less than 100 ppm, yet more preferably of less than 50 ppm, still more preferably of less than 20 ppm and most preferably of less than 10 ppm.

Because the dehydrobromination reaction is endothermic, it is preferred to add as heat vector an inert component to the mixture so as to bring energy to the reactor. Preferably the weight ratio of the one or more C₂₋₁₀-bromohydrocarbons and the inert component is preferably 1:10 to 10:1 and preferably 1:5 to 5:1. Suitable examples for the inert component are C1-10-alkanes, steam, nitrogen, argon, methane, carbon dioxide, naphtha and arbitrary combinations of two or more of the aforementioned inert compounds. Good results are in particular obtained, when the inert component is a C₃₋₇-alkane, more preferably a C₄₋₆-alkane and most preferably pentane.

Concerning the type of reactor, in which the dehydrobromination reaction is performed, the present invention is not particularly limited. Preferably, the reaction is performed in a fixed bed reactor, in a radial reactor, in a multitubular reactor, in a moving bed reactor or in a fluidized bed reactor. A suitable fluid bed reactor is one of the FCC types used for fluidized-bed catalytic cracking in the oil refinery. A typical moving bed reactor is of the continuous catalytic reforming type. Alternatively, the dehydrobromination reaction may be in a fixed bed reactor configuration using a pair of parallel "swing" reactors. This is possible on account of the high stability of the aforementioned catalyst.

it is proposed in a further development of the idea of the present invention that the dehydrobromination reaction is performed at an absolute pressure of 50 kPa to 3 MPa, more preferably of 50 kPa to 1 MPa, yet more preferably of 50 kPa to 500 kPa, still more preferably of 120 kPa to 500 kPa and most preferably of 120 kPa to 400 kPa. Preferably, the pressure is adjusted so that a partial pressure of the formed one or more olefinically unsaturated compounds, such as one or more C₂₋₁₀-alkenes of lower than 2 bars and more preferably of lower than 1 bar is achieved in the effluent of the reactor.

The dehydrobromination reaction is an endothermic reaction and thus requires the input of reaction heat in order to maintain the catalyst activity sufficiently high and in order to shift the thermodynamic equilibrium to sufficiently high reaction levels. In view of this, it is preferred that the dehydrobromination reaction is performed at a temperature of 200°C to 500°C, more preferably of 225°C to 450°C and most preferably of 250°C to 400°C.

In the case that the reaction is performed in a fluidized bed reactor using a stationary fluidized bed without catalyst circulation, the reaction temperature is substantially homogeneous throughout the catalyst bed. However, in the case that the reaction is performed in a fluidized bed reactor using a circulating fluidized bed so that the catalyst circulates between a reaction section and a catalyst regeneration section, the temperature in the catalyst bed approaches - depending on the degree of catalyst back mixing - either homogeneous conditions in case of a lot of back mixing or approaches plug flow conditions and hence a decreasing temperature profile will be formed as the reaction proceeds in case that nearly no back mixing occurs. In the case of performing the reaction in a fixed bed reactor or in a moving bed reactor, a decreasing temperature profile will form as the reaction of the proceeds. In this case reaction heat may be introduced by using several catalyst beds in series with inter heating of the reactor effluent from the first bed to higher temperatures and introducing the heated effluent in a second catalyst bed, etc. When the reaction is performed in a fixed bed reactor, a multi-tubular reactor may be used, in which the catalyst is loaded in small-diameter tubes that are installed in the reactor shell. At the shell side, a heating medium may be introduced that provides the required reaction heat by heat-transfer through the wall of the reactor tubes to the catalyst. A radial or spherical reactor may be used to overcome a too import pressure drop.

In accordance with a further preferred embodiment of the present invention, the weight hourly space velocity (WHSV) of the composition being catalytically reacted in the dehydrobromination reaction is 1 to 100 h⁻¹, more preferably 2 to 50 h⁻¹, still more preferably 3 to 20 h⁻¹ and most preferably 4 to 12 h⁻¹. Preferably, the weight hourly space velocity of the composition is measured as the ratio of the weight of the one or more C₂₋₁₀-bromohydro-carbons and the weight of the catalyst, without considering the diluent flow.

In a further development of the idea of the present invention, it is suggested that the catalyst is periodically regenerated by subjecting it in an oxygen containing atmosphere to a temperature of 400 to 525°C and preferably of 420 to 500°C. Thereby, the carbon on the catalyst is burnt with oxygen. In view of the noble and/or transition metals being contained in the catalyst, it is preferred to limit the exposition of the catalyst to a temperature of at most 525°C and preferably of 500°C. Preferably, the regeneration is performed at about 450°C with a temperature variation across the reactor of at most 50°C. This is typically achieved by limiting the amount of the oxygen in the regeneration loop at the initial stage to at most 1% by mole. After the removal of the main part of the carbon, the oxygen content may be raised to up to 5% by mole. Steam, hydrogen bromide and/or carbon dioxide may be present in the regeneration loop. After the end of the regeneration, it is preferred that all the traces of oxygen are removed from the catalyst, because the presence of oxygen catalyzes the rebromination of the C₂₋₁₀-alkane with hydrogen bromide.

Preferably, the composition containing hydrogen, hydrogen bromide and olefinically unsaturated compound is subjected to at least one separation step so as to separate it into a hydrogen bromide enriched composition and into an olefinically unsaturated compound enriched composition. Any suitable separation technique may be used, therefore. In accordance with a particular preferred embodiment of the present invention, the at least one separation step comprises at least one and preferably two distillation steps and/or comprises at least one absorption step.

In accordance with a particular preferred embodiment of the present invention, the at least one separation step comprises a (first) distillation step in which the composition containing hydrogen, hydrogen bromide and olefinically unsaturated compound is distilled, so as to separate it into a polybromohydrocarbon enriched composition as bottom composition and an overheads composition containing the olefinically unsaturated compound, hydrogen bromide and hydrogen.

Moreover, it is preferred that the at least one separation step comprises an absorption step, in which the composition containing hydrogen, hydrogen bromide and olefinically unsaturated compound is contacted with water or an aqueous solution so as to obtain a purified olefinically unsaturated compound enriched composition and a hydrogen bromide enriched composition.

Good results are in particular obtained, when the at least one separation step comprises a (first) distillation step in which the composition containing hydrogen, hydrogen bromide and olefinically unsaturated compound is distilled, so as to separate it into a polybromohydrocarbon enriched composition as bottom composition and an overheads composition containing the olefinically unsaturated compound, hydrogen bromide and hydrogen, and further comprises an absorption step, in which the overheads composition obtained in the distillation step is contacted with water or an aqueous solution so as to obtain a purified olefinically unsaturated compound enriched composition and a hydrogen bromide enriched composition. Good results are in particular obtained, when in the absorption step water or an aqueous composition is used as absorbing agent. The absorbing agent may contain at least 80% by weight and preferably at least 95% by weight of water and reminder to 100% by weight one or more compounds, such as, for instance, hydrogen bromide. It is particularly preferred that the aqueous composition used as absorbing agent contains hydrogen bromide, such as hydrogen bromide resulting from the recovery of the used absorbing agent, which is preferably used in circuit, i.e. recovered after the absorption step. In addition, the aqueous composition used as absorbing agent may comprise from its recovery, in particular after using an electrolysis step of converting hydrogen bromide to hydrogen and bromide and ternary column, more than 0 to 1,000 ppm of bromine, preferably 1 to 100 ppm of bromine and more preferably 1 to 10 ppm of bromine. In addition, it is preferred that the hydrogen bromide enriched composition has a hydrogen bromide concentration of at least 30% by weight and more preferably of at least 40% by weight as well as a content of absorbing agent of at most 60% by weight and more preferably of at most 48% by weight. Again, if the absorbing agent is water, in fact the hydrogen bromide enriched composition is an aqueous solution containing hydrogen bromide.

It is further preferred in this embodiment that the bottom composition obtained in the first distillation step is subjected to a further, second distillation step so as to obtain as overheads composition a light hydrocarbon composition and as bottom composition a polybromopropane enriched composition.

In a further development of the idea of the present invention, it is proposed that the hydrogen bromide enriched composition being obtained in the washing step is subjected to an electrolysis so as to obtain at one of the two electrodes hydrogen and at the other electrode a mixture containing bromine, water and hydrogen bromide, before the mixture containing hydrogen bromide, water and bromine is distilled so as to obtain as bottom product a mixture containing hydrogen bromide, water and more than 0 to 1,000 ppm of bromine, preferably 1 to 100 ppm of bromine and more preferably 1 to 10 ppm of bromine, which is recycled into the absorption step.

In addition, it is preferred that the at least one separation step comprises a pressure swing adsorption step so as to remove hydrogen from the olefinically unsaturated compound enriched composition or from the purified olefinically unsaturated compound.

The brominated hydrocarbon, such as a C₂₋₁₀-bromoalkane, for example bromo-propane, being included in the precursor composition, may be obtained by any suitable reaction. For instance, the brominated hydrocarbon may be obtained by reacting a mixture comprising the corresponding hydrocarbon, such as C₂₋₁₀-alkane, for example propane, and further comprising bromine so as to produce a reaction mixture containing brominated hydrocarbon, such as C₂₋₁₀-bromo-alkane, for example bromopropane, and hydrogen bromide. Good results are in particular obtained, when the molar ratio of the hydrocarbon to bromine in the mixture being reacted is 10:1 to 1:1 and preferably 3:1 to 2:1. Preferably, the reaction is performed without catalyst at a temperature of preferably 200 to less than 420°C, more preferably at a temperature of 230 to 410°C, still more preferably at a temperature of 240 to 400°C and most preferably at a temperature of 240 to 300°C. Moreover, it is preferred that the mixture is reacted at an elevated pressure, more preferably at a pressure of at least 400 kPa, still more preferably at a pressure of at least 1 MPa and most preferably at a pressure of 1 to 4 MPa. The elevated pressure leads to a high conversion rate per time unit and advantageously avoids or at least minimizes the formation of unsaturated products, such as of allyl bromide or propylene, in the reaction mixture. Good results are in particular obtained, when the residence time of the mixture in the reactor is at least 10 seconds and more preferably 20 seconds to 2 minutes. Residence time means in this connection the time period between the entrance of the mixture into the reactor, in which the reaction is performed, and the exit of the reaction mixture from the reactor. In practice, not all of the bromine and/or hydrocarbon will react during the bromination reaction to bromopropane, but also polybromopropanes will be formed and small amounts of bromine and propane will remain as non-reacted educts in the reaction mixture. Therefore, the reaction mixture obtained in the bromination reaction will in practice further contain one or more polybromopropanes, non-reacted propane and optionally at least traces of non-reacted bromine. Therefore, it is preferred to subject the reaction mixture to one or more separation steps, such as preferably one or two distillation steps and/or at least one absorption step with water or an aqueous absorption medium.

In accordance with another aspect the present invention relates to a plant comprising:
- a reactor for catalytically reacting one or more brominated hydrocarbons so as to produce a reaction mixture containing an olefinically unsaturated hydrocarbon and hydrogen bromide, wherein the reactor comprises an inlet line for the one or more brominated hydrocarbons and an outlet line for the reaction mixture,
- a separation unit comprising an inlet line for the reaction mixture being connected with the outlet line for the reaction mixture of the reactor, an outlet line for olefinically unsaturated hydrocarbon and an outlet line for a hydrogen bromide enriched composition,
wherein i) the inlet line for the one or more brominated hydrocarbons of the reactor is connected with an inlet line for hydrogen, or ii) the reactor comprises a further inlet line for hydrogen, or iii) the line connecting the outlet line for the reaction mixture of the reactor and the inlet line for the reaction mixture of the separation unit is connected with an inlet line for hydrogen, or iv) the separation unit is connected with an inlet line for hydrogen or v) an arbitrary combination of two or more of the aforementioned features i) to iv) is fulfilled.

Preferably, the separation unit comprises at least one and preferably two distillation columns and/or at least one absorption column.

Good results are in particular obtained, when the separation unit comprises:
- a first distillation column comprising an inlet line being connected with the outlet line for the reaction mixture, an overheads outlet line and a bottom outlet line,
- an absorption column comprising an inlet line being connected with the overheads outlet line of the first distillation column, an inlet line for absorbing agent, an outlet line for a hydrogen bromide enriched composition and an outlet line for a mixture of olefinically unsaturated compound and hydrogen and
- a second distillation column comprising an inlet line being connected with the bottom outlet line of the first distillation column, an overheads outlet line and a bottom outlet line.

In a further development of the idea of the present invention, it is suggested that the separation unit further comprises a separation device comprising an inlet line being connected with the outlet line for the mixture of olefinically unsaturated compound and hydrogen of the absorption column, an outlet line for hydrogen and an outlet line for olefinically unsaturated compound, wherein the separation device is preferably a pressure swing adsorption column.

Moreover, it is preferred that the plant further comprises a purification unit, wherein the purification unit comprises an electrolysis cell comprising an anode, a cathode and a membrane sandwiched between the anode and the cathode as well as an inlet line for a hydrogen bromide containing composition, an outlet line for hydrogen and an outlet line for a bromine containing composition, wherein the inlet line of the electrolysis cell is connected with the outlet line for the hydrogen bromide enriched composition of the separation unit, and wherein the outlet line for the bromine containing composition is connected with the inlet line of the reactor for catalytically reacting one or more brominated hydrocarbons.

In accordance with a further preferred embodiment of the present invention, the plant further comprises:
- a reactor for reacting a mixture comprising a hydrocarbon and bromine so as to produce a reaction mixture containing brominated hydrocarbon and hydrogen bromide, wherein the reactor comprises an inlet line for the mixture and an outlet line for the reaction mixture containing brominated hydrocarbon and hydrogen bromide,
- a first separation unit comprising an inlet line for the reaction mixture containing brominated hydrocarbon and hydrogen bromide being connected with the outlet line for the reaction mixture of the reactor for reacting a mixture comprising a hydrocarbon and bromine, an outlet line for a brominated hydrocarbon enriched composition and an outlet line for a hydrogen bromide enriched composition,
wherein the outlet line for a brominated hydrocarbon enriched composition is connected with the inlet line for the one or more brominated hydrocarbons of the reactor for catalytically reacting one or more brominated hydrocarbons.

Preferably, the separation unit comprises at least one and preferably two distillation columns and/or at least one absorption column.

Good results are in particular obtained, when the separation unit comprises:
- a first distillation column comprising an inlet line being connected with the outlet line for the reaction mixture of the reactor for reacting a mixture comprising a hydrocarbon and bromine, an overheads outlet line and a bottom outlet line,
- an absorption column comprising an inlet line being connected with the overheads outlet line of the first distillation column, an inlet line for absorbing agent, an outlet line for a hydrogen bromide enriched composition and an outlet line for a purified propane composition and
- a second distillation column comprising an inlet line being connected with the bottom outlet line of the first distillation column, an overheads outlet line for the bromopropane enriched composition and a bottom outlet line.

Specific embodiments in accordance with the present invention are subsequently described with reference to the appended drawing.

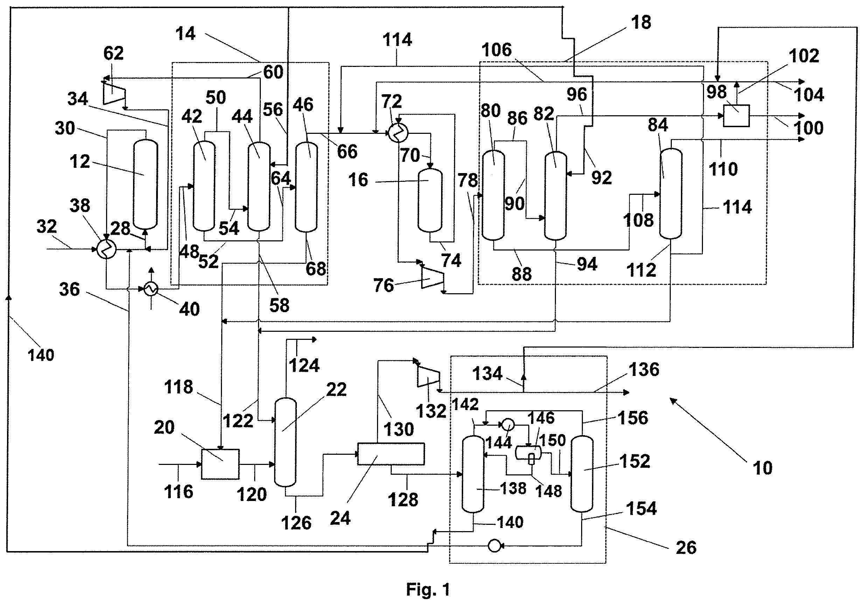

- Fig. 1: is a schematic view of a plant in accordance with one embodiment of the present invention.

The plant 10 shown in figure 1 and its operation is subsequently described using propane as specific example for a hydrocarbon, such as C₂₋₁₀-alkane, propylene as specific example for an olefinically unsaturated compound, such as C₂₋₁₀-alkene and bromopropane as specific example for a brominated hydrocarbon, such as C₂₋₁₀-bromoalkane. The plant 10 comprises:
i) a bromination reactor 12 for reacting a mixture comprising propane and bromine so as to produce a first reaction mixture containing bromopropane and hydrogen bromide,
ii) a first separation unit 14 for separating from the first reaction mixture a bromopropane enriched composition, a propane enriched composition, a polybromopropane enriched composition and a hydrogen bromide enriched composition,
iii) a dehydrobromination reactor 16 for catalytically reacting the bromopropane enriched composition being obtained in the first separation unit 14 so as to obtain a second reaction mixture containing propylene and hydrogen bromide,
iv) a second separation unit 18 for separating from the second reaction mixture propylene, a polybromopropane enriched composition and a hydrogen bromide enriched composition,
v) a thermal oxidizer 20 for oxidizing the polybromopropane enriched compositions being obtained in the first and second separation units,
vi) an absorption column 22 for contacting the hydrogen bromide enriched compositions being obtained in the first and second separation units and the oxidization product obtained in the thermal oxidizer 20,
vii) an electrolysis cell 24 for producing from the hydrogen bromide enriched composition being treated in the absorption column 22 hydrogen and a bromine containing composition and
viii) a purification unit 26 for obtaining pure bromine from the bromine containing composition being obtained in the electrolysis cell 24.

More specifically, the bromination reactor 12 comprises an inlet line 28 for the mixture comprising propane and bromine and an outlet line 30 for the first reaction mixture. The inlet line 28 is connected with a feed line 32 for a propane containing gas, such as natural gas, a recycle line 34 for propane and a recycle line 36 for bromine, wherein a heat exchanger 38 is arranged so that the first reaction mixture being withdrawn from the bromination reactor 12 via the outlet line 30 preheats the propane containing gas being led through the feed line 32. The outlet line 30 passes a further heat exchanger 40, before it leads into the first separation unit 14. The first separation unit 14 comprises, seen form the upstream to the downstream direction of the first separation unit 14, a first distillation column 42, an absorption column 44 and a second distillation column 46. While the first distillation column 42 comprises an inlet line 48 being connected with the outlet line 30 for the first reaction mixture of the bromination reactor 12, an overheads outlet line 50 and a bottom outlet line 52, the absorption column 44 comprises an inlet line 54 being connected with the overheads outlet line 50 of the first distillation column 42, an inlet line 56 for absorbing agent, an outlet line 58 for a hydrogen bromide enriched composition and an outlet line for the purified propane composition 60. The outlet line for the purified propane composition 60 leads via a compressor 62 into the recycle line 34 for propane. In turn, the second distillation column 46 comprises an inlet line 64 being connected with the bottom outlet line 52 of the first distillation column 42, an overheads outlet line 66 for the bromopropane enriched composition and a bottom outlet line 68 for a polybromopropane enriched composition.

The dehydrobromination reactor 16 for catalytically reacting the bromopropane enriched composition so as to produce a second reaction mixture containing propylene and hydrogen bromide comprises an inlet line 70 for the bromopropane enriched composition being connected via a heat exchanger 72 with the overheads outlet line 66 for bromopropane enriched composition of the second distillation column 46 of the first separation unit 14, and an outlet line 74 for the second reaction mixture, which leads via the heat exchanger 72 and a compressor 76 into the second separation unit 18. The second separation unit 18 comprises, seen form the upstream to the downstream direction of the second separation unit 18, a first distillation column 80, an absorption column 82 and a second distillation column 84. While the first distillation column 80 comprises an inlet line 78 for the second reaction mixture of the bromination reactor 12 being connected with the outlet line 74 of the dehydrobromination reactor 16, an overheads outlet line 86 and a bottom outlet line 88, the absorption column 82 comprises an inlet line 90 being connected with the overheads outlet line 86 of the first distillation column 80, an inlet line 92 for absorbing agent, an outlet line 94 for a hydrogen bromide enriched composition and an outlet line for the (purified) propylene 96. The outlet line for the (purified) propylene 96 leads into a pressure swing adsorption column 98, which comprises an outlet line 100 for propylene as well as an outlet line 102 for hydrogen splitting into a withdrawal line 104 for hydrogen and into a recycle line 106 leading back into the inlet line 70 for the second reaction mixture of the dehydrobromination reactor 16. In turn, the second distillation column 84 of the second separation unit 18 comprises an inlet line 108 being connected with the bottom outlet line 88 of the first distillation column 80, an overheads outlet line 110 for a C₄₋₅-hydrocarbon composition, a bottom outlet line 112 for a polybromopropane enriched composition and a recycle line 114 for a polybromopropane enriched composition leading into the overheads outlet line 66 of the second distillation column 44 of the first separation unit 14.

The oxidizing unit 20 comprises an inlet line 116 for air, an inlet line 118 being connected with the bottom outlet line 68 of the second distillation column 46 of the first separation unit 14 and being connected with the bottom outlet line 112 of the second distillation column 84 of the second separation unit 18, and an outlet line 120. In turn, the absorption column 22 comprises an inlet line being connected with the outlet line 120 of the oxidizing unit 20, an inlet line 122 for hydrogen bromide enriched composition being connected with the outlet line 58 for the hydrogen bromide enriched composition of the absorption column 44 of the first separation unit 14 and being connected with the outlet line 94 for the hydrogen bromide enriched composition of the absorption column 82 of the second separation unit 18, an outlet line 124 for air and an outlet line 126 for hydrogen bromide enriched composition being connected with the inlet line for hydrogen bromide containing composition of the electrolysis cell 24. The electrolysis cell 24 comprises an outlet line 128 for a bromine containing composition as well as an outlet line 130 for hydrogen, which is connected with a compressor 132, a recycle line 134 leading into the recycle line 106 and a hydrogen removal line 136. Finally, the purification unit 26 comprises a distillation column 138, which comprises an inlet line being connected with the outlet line 128 for the bromine containing composition of the electrolysis cell 24, a bottom outlet line and recycle line 140 for a mixture of water and hydrogen bromide and an overheads outlet line 142 for hydrogen. While the bottom outlet line and recycle line 140 of the purification unit 26 leads into the inlet lines 56, 92 for absorbing agent of the second distillation columns 44, 82 of the first and second separations units 14, 18, the overheads outlet line 142 leads into a condenser 144 and from there into a vessel 146, from which a return line 148 leads back into the distillation column 138 and an outlet line 150 leads into a dryer column 152. The dryer column 152 comprises a bottom outlet line for bromine, which is connected with the recycle line 36, and an overheads outlet 156, which leads back into the overheads outlet line 142 of the distillation column 138.

During the operation of the plant 10, a mixture comprising propane and bromine is led via line 28 into the bromination reactor 12, in which the propane and bromine react to bromopropane and hydrogen bromide. In addition, the (first) reaction mixture comprises non-reacted propane, non-reacted bromine and side-products, such as polybromopropanes. Then, the (first) reaction mixture is led via lines 30, 48 into the first distillation column 42 of the first separation unit, in which the reaction mixture is separated into a bottom composition mainly containing bromopropanes and minor amounts of polybromopropanes as well as into an overheads composition, which mainly contains hydrogen bromide, non-reacted propane and non-reacted bromine. The overheads composition is fed via lines 50, 54 into the absorption column 44 and is contacted there with absorbent agent, which is an aqueous solution containing minor amounts of hydrogen bromide recycled via lines 140, 56 into the absorption column 44, so as to obtain a purified propane composition, which is recycled into the mixture used in step a) via lines 60, 34 and the compressor 62, and a hydrogen bromide enriched composition being withdrawn from the absorption column 44 via line 58. The bottom composition of the first distillation column 42 of the first separation unit 14 mainly containing bromopropanes and minor amounts of polybromopropanes is led via 52, 64 into the second distillation column 46, in which the polybromopropanes are separated as bottom composition from the bromopropanes. While the polybromopropane enriched composition is withdrawn from the second distillation column 46 via line 68, the bromopropane enriched composition is withdrawn from the second distillation column 46 via line 66. Then, the bromopropane enriched composition is dehydro-brominated in the dehydrobromination reactor 16 in the presence of hydrogen so as to obtain a second reaction mixture containing propylene, hydrogen bromide, hydrogen and polybromopropanes. The second reaction mixture is then separated in the second separation unit 18 into pure propylene, which is withdrawn via line 100, into pure hydrogen, which is withdrawn via line 102 and partially recycled into the dehydrobromination reactor 16 via lines 102, 106, 70, into a polybromopropane enriched composition, which is withdrawn via line 112, and into a hydrogen bromide enriched composition, which is withdrawn via line 94. The two polybromopropane enriched compositions being withdrawn from the first and second separation units via lines 68, 112 are oxidized in the thermal oxidizer 20 mainly to bromine, which is then together with the two hydrogen bromide enriched compositions being withdrawn from the first and second separation units via lines 58, 94 contacted in the absorption column 22, from which via line 126 hydrogen bromide enriched composition (which is in fact an aqueous solution containing hydrogen bromide) is withdrawn and led into the electrolysis cell 24. The electrolysis cell 24 produces from this composition at the cathode hydrogen and at the anode bromine. While the hydrogen is withdrawn from the electrolysis cell 24 via line 130 and partially recycled into the dehydrobromination reactor 16 via lines 134, 106, 70, the bromine containing composition, which is an aqueous solution containing bromine and hydrogen bromide, is withdrawn from the electrolysis cell 24 via line 128. Afterwards, the bromine containing composition is separated in the distillation column 138 into an aqueous solution containing hydrogen bromide, which is withdrawn via line 140 and recycled via lines 56, 92 as absorbing agent into the absorption columns 44, 82 of the first and second separation units 14, 18, and into a bromine enriched composition. The latter is withdrawn from the distillation column 138 via line 142 and dewatered in the distillation column 152 so as to obtain pure bromine, which is led via lines 154, 36 into the mixture being introduced via line 28 into the bromination reactor 12.

The second reaction mixture contains propylene, hydrogen bromide, hydrogen and polybromopropanes, wherein the hydrogen protects the propylene from being rebrominated by the hydrogen bromide from the generation of the second reaction mixture and during the whole separation steps until the hydrogen is removed from the purified propylene. In the embodiment shown in figure 1, the hydrogen is added to a precursor composition of the reaction mixture, namely into the bromopropane enriched composition being withdrawn from the distillation column 46 via the overheads outlet line 66.

### Examples

### Control

An empty quartz tube with a length of 50 cm and an internal diameter of 10 mm was covered with aluminum foil to avoid light penetration. The tube was heated to 120°C. The entrance of the reactor was connected to on-line to GC analysis.

A blank test was performed with 30 ml/min of He, 3 ml/min of propylene and 2 ml/min of nitrogen for 10 min at atmospheric pressure during 2 hours on stream at 120°C. The total gas flow (i.e. flow of hydrogen bromide, propylene and nitrogen) to the reactor was 35 ml/min. No changes in the reaction mixture were observed.

### Comparative Example 1

The experiment was repeated under the same conditions as set out above for the control except that the He flow was replaced by an equal hydrogen bromide flow (i.e. of 30 ml/min), whereas the flow of propylene was remained at 3 ml/min and the flow of nitrogen was remained at 2 ml/min. The total gas flow (i.e. flow of hydrogen bromide, propylene and nitrogen) to the reactor was 35 ml/min at atmospheric pressure during 2 hours on-stream at 120°C. The molar ratio hydrogen bromide / propylene was about 10. About 55% of propylene was converted to bromopropane, i.e. rebrominated.

### Comparative Example 2

The Comparative Example 1 was repeated except that the hydrogen bromide flow was changed to 6 ml/min, the propylene flow rate was remained at 3 ml/min and the nitrogen flow rate was changed to 26 ml/min. The total gas flow (i.e. flow of hydrogen bromide, propylene and nitrogen) to the reactor was 35 ml/min at atmospheric pressure during 2 hours on-stream at 120°C. The molar ratio hydrogen bromide/propylene was about 2. About 12% of propylene was converted to bromopropane, i.e. rebrominated. A lower amount of rebromination was observed in comparison to Comparative Example 1 due to lower partial pressure of hydrogen bromide (i.e. lower molar ratio of hydrogen bromide / propylene).

### Comparative Example 3

The Comparative Example 2 was repeated except that with a hydrogen bromide flow rate of 6 ml/min, a propylene flow rate of 3 ml/min and a nitrogen flow rate of 26 ml/min were adjusted. The total gas flow (i.e. flow of hydrogen bromide, propylene and nitrogen) to the reactor was 35 ml/min at atmospheric pressure during 2 hours on-stream at 120°C. The molar ratio hydrogen bromide / propylene was about 2. Bromopropane has reappeared in the effluent mixture. About 9% of propylene was again converted to bromopropane, i.e. rebrominated.

### Example 1

The Comparative Example 1 except that a hydrogen bromide flow rate of 6 ml/min, a propylene flow rate of 3 ml/min, a hydrogen flow rate of 6 ml/min and a nitrogen flow rate of 20 ml/min were adjusted. The total gas flow (i.e. flow of hydrogen bromide, propylene and nitrogen) to the reactor was 35 ml/min at atmospheric pressure during 2 hours on-stream at 120°C. The molar ratio hydrogen bromide / propylene was about 2. No bromopropane was detected.

### Reference Numeral List

- 10: Plant
- 12: Bromination reactor
- 14: First separation unit
- 16: Dehydrobromination reactor
- 18: Second separation unit
- 20: Thermal oxidizer
- 22: Absorption column
- 24: Electrolysis cell
- 26: Purification unit
- 28: Inlet line for the first reaction mixture of the bromination reactor
- 30: Outlet line of the reactor
- 32: Feed line for a propane containing gas
- 34: Recycle line for propane
- 36: Recycle line for bromine
- 38: Heat exchanger
- 40: Heat exchanger
- 42: First distillation column of the first separation unit
- 44: Absorption column of the first separation unit
- 46: Second distillation column of the first separation unit
- 48: Inlet line of the first distillation column of the first separation unit
- 50: Overheads outlet line of the first distillation column
- 52: Bottom outlet line of the first distillation column
- 54: Inlet line of the absorption column
- 56: Inlet line for absorbing agent
- 58: Outlet line for a hydrogen bromide enriched composition
- 60: Outlet line for a purified propane composition
- 62: Compressor
- 64: Inlet line of the second distillation column
- 66: Overheads outlet line of the second distillation column
- 68: Bottom outlet line of the second distillation column
- 70: Inlet line for the second reaction mixture of the dehydrobromination reactor
- 72: Heat exchanger
- 74: Outlet line for second reaction mixture
- 76: Compressor
- 78: Inlet line of the first distillation column of the second separation unit
- 80: First distillation column of the second separation unit
- 82: Absorption column of the second separation unit
- 84: Second distillation column of the second separation unit
- 86: Overheads outlet line of the first distillation column
- 88: Bottom outlet line of the first distillation column
- 90: Inlet line of the absorption column
- 92: Inlet line for absorbing agent
- 94: Outlet line for a hydrogen bromide enriched composition
- 96: Outlet line for purified propylene
- 98: Pressure swing adsorption column
- 100: Outlet line for propylene
- 102: Outlet line for hydrogen
- 104: Withdrawal line for hydrogen
- 106: Recycle line for hydrogen
- 108: Inlet line of the second distillation column of the second separation unit
- 110: Overheads outlet line for a C₄₋₅-hydrocarbon composition
- 112: Bottom outlet line of the second distillation column of the second separation unit
- 114: Recycle line for polybromopropane enriched composition
- 116: Inlet line for air
- 118: Inlet line for polybromopropane enriched composition
- 120: Outlet line of thermal oxidizer
- 122: Inlet line for hydrogen bromide enriched composition
- 124: Outlet line for air
- 126: Outlet line for hydrogen bromide enriched composition
- 128: Outlet line for bromine containing composition
- 130: Outlet line for hydrogen
- 132: Compressor
- 134: Recycle line for hydrogen
- 136: Hydrogen removal line
- 138: Distillation column of purification unit
- 140: Bottom outlet line for a mixture of water and hydrogen bromide
- 142: Overheads outlet line for hydrogen
- 144: Condenser
- 146: Vessel
- 148: Return line
- 150: Outlet line of vessel
- 152: Dryer column
- 154: Bottom outlet of dryer column
- 156: Overheads outlet of dryer column

## Claims

1. A process for mitigating a reaction of hydrogen bromide with one or more olefinically unsaturated compounds comprising i) the step of adding hydrogen to a composition containing hydrogen bromide and olefinically unsaturated compound so as to obtain a composition containing hydrogen, hydrogen bromide and olefinically unsaturated compound and/or ii) the step of adding hydrogen to a precursor composition, from which a composition containing hydrogen, hydrogen bromide and olefinically unsaturated compound is formed.

2. The process in accordance with claim 1, wherein the composition containing hydrogen, hydrogen bromide and olefinically unsaturated compound also comprises bromine and/or the precursor composition, from which a composition containing hydrogen, hydrogen bromide and olefinically unsaturated compound is formed, also comprises bromine.

3. The process in accordance with claim 1 or 2, wherein the molar concentration of the hydrogen in the composition containing hydrogen, hydrogen bromide and olefinically unsaturated compound is adjusted to at least 0.1% by mol, preferably 0.2 to 90% by mol and more preferably to 0.5 to 60% by mol.

4. The process in accordance with any of the preceding claims, wherein the molar ratio of hydrogen to olefinically unsaturated compound in the composition containing hydrogen, hydrogen bromide and olefinically unsaturated compound is adjusted to be 2:1 to 1:10, and/or, wherein the molar ratio of hydrogen to the hydrogen bromide in the composition containing hydrogen, hydrogen bromide and olefinically unsaturated compound is adjusted to be 2:1 to 1:5.

5. The process in accordance with any of the preceding claims, wherein the olefinically unsaturated compound is an olefinically unsaturated hydrocarbon, preferably a C₂₋₁₀-alkene, more preferably a C₂₋₅-monoalkene or a C₄₋₈-dialkene, still more preferably ethylene or propylene and most preferably propylene.

6. The process in accordance with any of the preceding claims, wherein the hydrogen is added to a composition containing 1 to 90% by weight and preferably 20 to 50% by weight of hydrogen bromide and 20 to 90% by weight and preferably 30 to 50% by weight of one or more olefinically unsaturated compounds.

7. The process in accordance with any of claims 1 to 4, wherein the hydrogen is added to a precursor composition, which comprises one or more brominated hydrocarbons, before the so obtained mixture is subjected to a dehydrobromination reaction so as to form the composition containing hydrogen, hydrogen bromide and one or more olefinically unsaturated compounds, wherein preferably the precursor composition contains one or more C₂₋₁₀-bromohydrocarbons, preferably C₂₋₁₀-bromo-alkanes, more preferably C₂₋₅-monobromoalkanes or C₄₋₈-dibromoalkanes, still more preferably bromoethane or bromopropane and most preferably bromopropane.

8. The process in accordance with claim 6 or 7, wherein the hydrogen is added to the precursor composition so that the molar ratio of the one or more brominated hydrocarbons and the hydrogen in the obtained mixture is 1:10 to 10:1 and preferably 1:5 to 5:1.

9. The process in accordance with claim 7 or 8, wherein the mixture is subjected to a catalytic dehydrobromination reaction, wherein the catalyst comprises a support material and thereon at least 200 ppm of at least one metal being selected from the group consisting of transition metals, noble metals and arbitrary combinations of two or more thereof.

10. The process in accordance with any of the preceding claims, wherein the composition containing hydrogen, hydrogen bromide and olefinically unsaturated compound is subjected to at least one separation step so as to separate it into a hydrogen bromide enriched composition and into an olefinically unsaturated compound enriched composition, wherein preferably the at least one separation step comprises a distillation step in which the composition containing hydrogen, hydrogen bromide and olefinically unsaturated compound is distilled, so as to separate it into a polybromohydrocarbon enriched composition as bottom composition and an overheads composition containing the olefinically unsaturated compound, hydrogen bromide and hydrogen.

11. The process in accordance with claim 10, wherein the at least one separation step comprises an absorption step, in which the composition containing hydrogen, hydrogen bromide and olefinically unsaturated compound or the overheads composition obtained in the distillation step is contacted with water or an aqueous solution so as to obtain a purified olefinically unsaturated compound enriched composition and a hydrogen bromide enriched composition, wherein the water or aqueous solution contains more than 0 to 1,000 ppm of bromine, preferably 1 to 100 ppm of bromine and more preferably 1 to 10 ppm of bromine, and wherein preferably the hydrogen bromide enriched composition is subjected to an electrolysis so as to obtain at one of the two electrodes hydrogen and at the other electrode a mixture containing bromine, water and hydrogen bromide, before the mixture containing hydrogen bromide, water and bromine is distilled so as to obtain as bottom product a mixture containing hydrogen bromide, water and more than 0 to 1,000 ppm of bromine, preferably 1 to 100 ppm of bromine and more preferably 1 to 10 ppm of bromine, which is recycled into the absorption step.

12. A plant comprising:
- a reactor for catalytically reacting one or more brominated hydrocarbons so as to produce a reaction mixture containing an olefinically unsaturated hydrocarbon and hydrogen bromide, wherein the reactor comprises an inlet line for the one or more brominated hydrocarbons and an outlet line for the reaction mixture,
- a separation unit comprising an inlet line for the reaction mixture being connected with the outlet line for the reaction mixture of the reactor, an outlet line for olefinically unsaturated hydrocarbon and an outlet line for a hydrogen bromide enriched composition,
wherein i) the inlet line for the one or more brominated hydrocarbons of the reactor is connected with an inlet line for hydrogen, or ii) the reactor comprises a further inlet line for hydrogen, or iii) the line connecting the outlet line for the reaction mixture of the reactor and the inlet line for the reaction mixture of the separation unit is connected with an inlet line for hydrogen, or iv) the separation unit is connected with an inlet line for hydrogen or v) an arbitrary combination of two or more of the aforementioned features i) to iv) is fulfilled.

13. The plant in accordance with claim 12, wherein the separation unit comprises at least one and preferably two distillation columns and/or at least one absorption column.

14. The plant in accordance with claim 12 or 13, which further comprises a purification unit, wherein the purification unit comprises an electrolysis cell comprising an anode, a cathode and a membrane sandwiched between the anode and the cathode as well as an inlet line for a hydrogen bromide containing composition, an outlet line for hydrogen and an outlet line for a bromine containing composition, wherein the inlet line of the electrolysis cell is connected with the outlet line for the hydrogen bromide enriched composition of the separation unit, and wherein the outlet line for the bromine containing composition is connected with the inlet line of the reactor for catalytically reacting one or more brominated hydrocarbons.

15. The plant in accordance with claim 12 or 13, which further comprises:
- a reactor for reacting a mixture comprising a hydrocarbon and bromine so as to produce a reaction mixture containing brominated hydrocarbon and hydrogen bromide, wherein the reactor comprises an inlet line for the mixture and an outlet line for the reaction mixture containing brominated hydrocarbon and hydrogen bromide,
- a first separation unit comprising an inlet line for the reaction mixture containing brominated hydrocarbon and hydrogen bromide being connected with the outlet line for the reaction mixture of the reactor for reacting a mixture comprising a hydrocarbon and bromine, an outlet line for a brominated hydrocarbon enriched composition and an outlet line for a hydrogen bromide enriched composition,
wherein the outlet line for a brominated hydrocarbon enriched composition is connected with the inlet line for the one or more brominated hydrocarbons of the reactor for catalytically reacting one or more brominated hydrocarbons.
